(19) Europäisches Patentamt
European Patent Office
Office européen des brevets

(11) **EP 1 173 757 B1**

(12) **EUROPEAN PATENT SPECIFICATION**

(45) Date of publication and mention
of the grant of the patent:
**06.08.2008 Bulletin 2008/32**

(51) Int Cl.:
***G01N 33/50*** (2006.01)

(21) Application number: **00928274.0**

(86) International application number:
**PCT/US2000/010775**

(22) Date of filing: **21.04.2000**

(87) International publication number:
**WO 2000/065346 (02.11.2000 Gazette 2000/44)**

(54) **DIAGNOSIS OF HUMAN KIDNEY DISEASES**

DIAGNOSTISCHES VERFAHREN FÜR NIERENKRANKHEITEN IN MENSCHEN

DIAGNOSTIC DE MALADIES DU REIN CHEZ L'HUMAIN

(84) Designated Contracting States:
**AT BE CH CY DE DK ES FI FR GB GR IE IT LI LU MC NL PT SE**

(30) Priority: **23.04.1999 US 130903 P**
**23.04.1999 US 130908 P**

(43) Date of publication of application:
**23.01.2002 Bulletin 2002/04**

(73) Proprietor: **Shiva Biomedical, LLC**
**Paramus, NJ 07652 (US)**

(72) Inventor: **SHAH, Sudhir, V.**
**Little Rock, AR 72223 (US)**

(74) Representative: **Kirkham, Nicholas Andrew et al**
**Graham Watt & Co LLP**
**St Botolph's House**
**7-9 St Botolph's Road**
**Sevenoaks**
**Kent TN13 3AJ (GB)**

(56) References cited:
WO-A-00/13706          WO-A-91/07956
WO-A-94/01770          DE-A- 4 008 323

- ZHA-LONG WU AND MARK S. PALLER: "IRON LOADING ENHANCES SUSCEPTIBILITY TO RENAL ISCHEMIA IN RATS" RENAL FAILURE, vol. 16, no. 4, 1994, pages 471-480, XP000916923
- BALIGGA RADHAKRISHNA ET AL: "iN VITRO AND IN VIVO EVIDENCE SUGGESTING A ROLE FOR IRON IN CISTAPLIN-INDUCED NEPHROTOXICITY" KIDNEY INTERNATIONAL, vol. 53, no. 2, February 1998 (1998-02), pages 394-401, XP000916879
- HARRIS DAVID C H AND TAY YUET-CHING: "MITOCHONDRIAL FUNCTION IN RAT RENAL CORTEX IN RESPONSE TO PROTEINURIA AND IRON" CLINICAL AND EXPERIMENTAL PHARMACOLOGY AND PHYSIOLOGY, vol. 24, December 1997 (1997-12), pages 916-922, XP000916876 AUSTRALIA

**Description**

BACKGROUND OF THE INVENTION

Kidney disease can be progressive, leading to end-stage renal disease and, ultimately, death. End-stage renal disease affects approximately 650,000 human patients each year worldwide and treatment costs have been estimated to be about twenty billion dollars (Klahr, S., Contrib. Nephrol. 118:1-5 (1996)).

Current methods to diagnose kidney disease and, in particular, progressive kidney disease, include monitoring urine for elevated protein levels and conducting renal biopsies. Available treatments for kidney disease, specifically progressive kidney disease, include the use of steroids, alkylating agents and cyclosporine (de Mattos, A.M. et al., pages 861-885, In: "Immunologic Renal Diseases," eds. Neilson, E.G. et al., Lippincott-Raven Publishers, Philadelphia, PA (1997)). The above mentioned diagnostic methods often are inadequate since significant damage to the kidney can occur prior to diagnosis. Likewise, the above mentioned treatments frequently are unsuccessful in halting the progression of kidney disease and, therefore, unsatisfactory, since they often are accompanied by adverse side effects, such as cellular and systemic toxicity.

WO 94/01770 discloses a method of computer urine diagnostics for kidney diseases.

DE 4,008,323 discloses the use of gallopamil for the production of pharmaceuticals for the treatment of acute kidney failure.

Although animal models have been used to study some kidney diseases, animal models suffer from a number of limitations when extended to human kidney disease diagnosis and treatment. For example, animal models of kidney disease do not necessarily depict kidney disease in humans and the administration of steroids, although reasonably effective in the treatment of kidney disease in experimental animal models, are relatively ineffective in the treatment of kidney disease in humans (Siegel, N.J. et al., Kidney Int. 25:906-911 (1984); Cronin, R.E., et al., Am. J. Physiol. 248: F332-F339 (1985); Cronin, R.E., et al., Am. J. Physiol. 251:F408-F416 (1986); Seiken, G. et al., Kidney Int. 45:1622-1627 (1994); Shaw, S.G. et al., J. Clin. Invest. 80:1232-1237 (1987); Allgren, R.L. et al., N. Eng. J. Med. 336:828-834 (1997); Paller, M..S., Sem. Nephrol. 18:482-489 (1998); Savill, J., et al., In: "Oxford Textbook of Clinical Nephrology", eds. Davison, A.M., et al.,, Oxford Medical Publications, page 403-439 (1998); Hirschberg, R. et al., Kidney Int. 55:2423-2432 (1999)). Thus, animal model systems generally can not be used to define and identify methods which halt the progression, reduce the severity, treat, or diagnose progressive kidney disease in humans. Thus, there is a need to develop new, improved and effective methods of reducing the severity, diagnosing and treating kidney disease in humans.

SUMMARY OF THE INVENTION

The present invention relates to methods of diagnosing and treating kidney disease in a human. In particular, the invention relates to methods of treating progressive kidney diseases.

In one embodiment, the method includes diagnosing a kidney disease in a human comprising the step of measuring catalytic iron in a urine sample. In a preferred embodiment, the catalytic iron is measured in relation to a reference protein. In a particularly preferred embodiment, the reference protein is urinary creatinine.

In another embodiment, the invention relates to a method of treating a kidney disease in a human comprising measuring catalytic iron content in a urine sample obtained from the human and comparing catalytic iron content in the urine sample with catalytic iron content in a control sample. A catalytic iron content in the urine sample above catalytic iron content in the control sample is indicative of kidney disease.

In yet another embodiment, the invention includes a method of treating a kidney disease in a human, wherein the human has a catalytic iron content in urine greater than about 15 nmol/mg of a reference protein comprising administering an iron chelator to the human.

In an additional embodiment, the invention includes a method of treating a kidney disease in a human comprising administering a dose of an iron chelator to a human having catalytic iron content in urine exceeding that of a control sample and obtaining a first urine sample from the human. The catalytic iron content in the first urine sample is measured and compared to the catalytic iron content in the catalytic iron content in the control sample.

The method can further include administering at least one subsequent dose of the iron chelator. The amount of the subsequent dose is determined by comparison of the measured catalytic iron content in the urine sample to the catalytic iron content in the control sample.

In another embodiment, the method further includes obtaining at least one subsequent urine sample and measuring catalytic iron content in the subsequent urine sample. The catalytic iron content in the subsequent urine sample is compared to the catalytic iron content in the first urine sample, the control sample or both.

Yet another embodiment of the invention includes determining progression of kidney disease in a human. Catalytic iron content in a first urine sample and at least one additional urine sample obtained from the human is measured. Catalytic iron content in the additional urine sample is compared with catalytic iron content in the first urine sample. An elevation in catalytic iron content in an additional urine sample above catalytic iron content in the first urine sample is indicative of the progression of the kidney disease in the human.

In still another embodiment, the invention relates to a method of evaluating the effectiveness of administering an iron chelator to treat a human suffering from a progressive kidney disease. Catalytic iron content in a first urine sample obtained from the human is compared with catalytic iron content of a subsequent urine sample obtained from the human. The subsequent urine sample is obtained after obtaining the first urine sample and after the administration of the iron chelator. A comparison of the catalytic iron content in the subsequent urine sample and the first urine sample is indicative of the effectiveness of the administration of the iron chelator to alleviate progression of the kidney disease.

In another embodiment, the invention relates to a method of identifying a human suffering from a progressive kidney disease who will benefit from treatment of the progressive kidney disease with the iron chelator. Catalytic iron content in a urine sample obtained from the human is measured. A catalytic iron content in the urine sample greater than about 15 nmol/mg of a reference protein identifies a human suffering from a progressive kidney disease who will benefit from treatment of the progressive kidney disease with the iron chelator.

In yet another embodiment, the invention relates to a method of treating microalbuminuria in a human comprising administering a dose of an iron chelator to a human suffering from microalbuminuria having a catalytic iron content in urine exceeding a control sample. A first urine sample is obtained from the human and catalytic iron content measured in the first urine sample. The catalytic iron content in the first urine sample is compared with the catalytic iron content in the control sample.

The invention described herein provides methods for diagnosing, treating, halting the progression and reducing the severity of kidney disease in a human. Advantages of the claimed invention include, for example, treatment of progressive kidney disease in a human in a manner which has not been described in an animal model, nor could not be predicted from existing animal models of kidney disease. The methods of the invention can treat progressive kidney diseases in humans without significant side effects. The methods of the invention provide an effective manner to treat kidney disease and, ultimately, prevent end-stage renal disease.

Thus, treatment of humans who have a catalytic iron content in the urine above a control level with iron chelators, can halt, reverse, or reduce the severity of the progression of kidney disease, thereby increasing quality of life and life expectancy.

BRIEF DESCRIPTION OF THE FIGURES

The Figure represents the catalytic iron content (nmol/mg urinary creatinine) in urine obtained from humans with no kidney disease (control), with diabetic microalbuminuria (DM Micro), diabetic (DM) proteinuria, glomerulonephritis (GN) and ischemic nephrophathy.

DETAILED DESCRIPTION OF THE INVENTION

The features and other details of the invention, either as steps of the invention or as combinations of parts of the invention, will now be more particularly described and pointed out in the claims. It will be understood that the particular embodiments of the invention are shown by way of illustration and not as limitations of the invention. The principle features of this invention can be employed in various embodiments without departing from the scope of the invention.

The present invention relates to the discovery that catalytic iron in urine of a human can be an indicator of the existence of kidney disease. The invention further relates to the discovery that the administration of an iron chelator decreases protein content in urine of humans suffering from kidney diseases. In particular, the iron chelator deferiprone has been found to decrease protein content and serum creatinine, both indices of kidney damage, in urine obtained from humans suffering from progressive kidney disease.

"Kidney disease" generally refers to a disorder of at least one kidney in a human that compromises the function of the kidney (e.g., to prevent leakage of protein, to prevent excretion of nitrogenous waste). The kidney disease can result from a primary pathology of the kidney (e.g., injury to the glomerulus or tubule) or another organ (e.g., pancreas) which adversely affects the ability of the kidney to perform biological functions (e.g., retain protein). Thus, kidney disease in the human can be the direct or indirect effect of disease. Examples of a kidney disease as a result or consequence of an indirect effect on the kidneys is kidney disease as a consequence of diabetes or systemic lupus. The term kidney disease is used interchangeably with the phrase "diseases of the kidney." The kidney disease can be, for example, a result or a consequence of any change, damage, or trauma to the glomerulus, tubules or interstitial tissue in either the renal cortex or renal medulla of the kidney.

In a preferred embodiment of the invention, the kidney disease is a progressive kidney disease. "Progressive kidney disease" as used herein refers to any disease of the kidney that over time (e.g., days, weeks, months, years) leads to a loss of renal function. "Renal function" generally refers to a physiological property of the kidney, such as the ability to retain protein thereby preventing proteinuria (e.g., urinary creatinine, the excretion of protein in an amount greater than about 0.15g/24 hours). Renal function can be assessed, for example, by glomerular filtration rate (e.g., creatinine clearance), excretion of protein in urine, blood urea nitrogen, serum or plasma creatinine, or any combination thereof.

Glomerular filtration rate is an indicia of renal function which generally refers to the renal (also referred to herein as kidney) excretory capacity. Indices to assess glomerular filtration include, for example, creatinine clearance and inulin clearance. Exemplary descriptions and discussions of techniques to assess renal function, including glomerular filtration rate (e.g., creatinine clearance) are found, for example, in Larsen, K. Clin. Chem. Acta. 41:209-217 (1972); Talke, H. et al., Klin, Wscr 41:174 (1965); Fujita, Y. et al., Bunseki Kgaku 32:379-386 (1983); Rolin, H.A. III, et al., In: "The Principles and Practice of Nephrology", 2nd ed., Jacobson, H.R., et al., Mosby-Year Book, Inc., St. Louis, MO, page 8-13 (1995); Carlson, J.A., et al., In: "Diseases of the Kidney", 5th Ed., Schrier, R.W. et al., eds., Little, Brown and Co., Inc., Boston, MA, pages 361-405 (1993). For example, endogenous creatinine clearance can be determined as follows:

$$Cr = Ucr \ V/PCr$$

where Cr = clearance of creatinine (ml/min); Ucr = urine creatinine (mg/dl), V = volume of urine (ml/min - for 24-hr volume: divide by 1400, and PCr = plasma creatinine (mg/dl).

A progressive kidney disease treated by the methods described herein includes any kidney disease that can, ultimately, lead to end-stage renal disease. A progressive kidney disease of the invention is not a disease of the kidney that results from exogenous iron overload as a result of, for example, repeated blood transfusions in diseases such as thalassemia or sickle cell anemia. The progressive kidney diseases that can be diagnosed or treated by the methods of the invention can be, for example, associated with endogenous iron deposit in the kidney (e.g., glomerulus, tubules). The endogenous iron can be released, for example, from ferritin, mitochondria or cytochrome P450 in the human.

In a more preferred embodiment, the kidney disease is a progressive glomerular kidney disease. Progressive glomerular kidney diseases that are particularly suitable for treatment by the method of the invention include, for example, diabetic nephropathy (e.g., as a consequence of Type I or Type II diabetes or systemic lupus), primary glomerulonephritis (e.g., membranous nephropathy, focal segmental glomerulosclerosis, membranoproliferative glomerulonephritis, diffuse proliferative glomerulonephritis, membranous focal segmental glomerulosclerosis) and secondary glomerulonephritis (e.g., diabetic nephropathy, ischemic nephropathy).

A kidney disease can be diagnosed in a human by the method of the invention by measuring the catalytic iron in urine of the human. A human is also referred to herein as a patient or an individual. In a preferred embodiment, a human can be diagnosed with a kidney disease when catalytic iron content in urine is greater than about 15 nmol/mg of a reference protein.

"Catalytic iron" refers to $Fe^{2+}$. Catalytic iron is capable of catalyzing free radical reactions. Iron in the $Fe^{2+}$ state can catalyze the Haber-Weiss reaction, which reduces hydrogen peroxide and promotes formation of hydroxyl radicals. The Haber-Weiss reaction is illustrated below:

$$\underline{O}_2^- + Fe^{3+} \rightarrow O_2 + Fe^{2+}$$

$$Fe^{2+} + H_2\underline{O}_2 \rightarrow Fe^{3+} + OH\bullet + OH^-$$

$$\underline{O}_2^- + H_2O_2 \rightarrow O_2 + OH\bullet + OH^-$$

It is also envisioned that catalytic iron can result in the formation of hydroxyl radicals in reactions other than the Haber-Weiss reaction.

Methods to determine the catalytic iron content are known (see, for example, Gutteridge, J.M.C. et al., Biochem. J. 199:263-265 (1981); Yergey, A.L. J. Nutrition 126:355S-361S (1996); Iancu, T.C. et al., Biometals 9:57-65 (1996); Artiss, J.D. et al., Clin. Biochem. 14:311-315 (1981); Smith, F.E. et al., Clin. Biochem. 17:306-310 (1984); Artiss, J.D., et al., Microchem. J. 28:275-284 (1983)) and as described in the exemplification. The methods include spectrophotometric, mass spectrometry (e.g., thermal ionization mass spectrometry, laser microprobe mass analysis, inductively coupled plasma mass spectrometry, atom bombardment-secondary ion mass spectrometry). Catalytic iron content is also referred to as an amount, level or concentration of catalytic iron.

In a preferred embodiment, the catalytic iron content is measured in relation to a reference protein in the urine sample. The phrase "in relation to a reference protein" as used herein refers to expressing catalytic iron content in urine as a unit of measurement (e.g., nanomoles) per a concentration of a protein (e.g., milligrams) in the urine. Catalytic iron can be expressed, for example, as nanomoles (nmoles) of catalytic iron per milligram (mg) of protein in urine. In a preferred embodiment, the reference protein is urinary creatinine.

Additionally, or alternatively, catalytic iron content is measured in relation to glomerular filtration rate, in particular creatinine clearance as described above.

Preferably, catalytic iron content is measured in a urine sample from a human prior to the onset of clinical symptoms (also referred to herein as clinical signs of kidney disease) of kidney disease (e.g., proteinuria, increase blood urea nitrogen, increase serum creatinine). Additionally, or alternatively, catalytic iron content is measured in a urine sample obtained from the human after the onset of clinical symptoms of kidney disease (e.g., proteinuria). Catalytic iron is measured in humans afflicted with a kidney disease and compared to humans not afflicted with a kidney disease (See Tables 1 and 2).

An "iron chelator" refers to any molecule capable of interacting with iron, either $Fe^{3-}$ or $Fe^{2+}$, to prevent the formation of catalytic iron from $Fe^{3+}$ or to prevent, inhibit or interfere with iron ($Fe^{3-}$ or $Fe^{2+}$) interacting, effecting or participating in the Haber-Weiss reaction or any other reaction which can generate hydroxyl radicals. The interaction between the iron chelator and iron, either $Fe^{3+}$, $Fe^{2+}$, or both, can be, for example, a binding interaction, an interaction as a result of steric hindrance or any reciprocal effect between iron and the iron chelator. The iron chelator can, for example, prevent the conversion of $Fe^{3+}$ to $Fe^{2+}$, thereby indirectly preventing the reduction of hydrogen peroxide and formation of hydroxyl radicals in the Haber-Weiss reaction. Alternatively, or additionally, the iron chelator can interact directly with $Fe^{2+}$ to prevent hydroxyl radical formation in, for example, the Haber-Weiss reaction.

The iron chelator can be a peptide comprising natural or nonnatural (e.g., amino acids not found in nature) amino acids, polyethylene glycol carbamates, lipophilic or nonlipophilic polyaminocarboxylic acids, polyanionic amines or substituted polyaza compounds. In a preferred embodiment, the iron chelator is deferiprone (1,2-dimethyl-3-hydroxy-pyrid-4-one)L1. Iron chelators are commercially available or can be synthesized or purified from biological sources using routine procedures. Exemplary descriptions and discussions of iron chelators are found in several references, for example, U.S. Patent No: 5,047,421 (1991); U.S. Patent No: 5,424,057 (1995); U.S. Patent No: 5,721,209 (1998); U.S. Patent No: 5,811,127 (1998); Olivieri, N.F. et al., New Eng. J. Med. 332:918-922 (1995); Boyce, N.W. et al., Kidney International. 30:813-817 (1986); Kontoghiorghes, G.J. Indian J. Pedirr. 60:485-507 (1993); Hershko, C. et al., Brit. J. Haematology 101:399-406 (1998); Lowther, N. et al., Pharmac. Res. 16:434 (1999).

An "amount effective," when referring to the amount of iron chelator is defined as that amount (also referred to herein as dose) of iron chelator that, when administered to a human afflicted with a kidney disease, is sufficient for therapeutic efficacy (e.g., an amount sufficient to reduce the catalytic iron content in urine, total protein content in urine, blood urea nitrogen, or creatinine in a blood sample obtained from a human with a progressive kidney disease). An effective amount of an iron chelator also refers to an amount of iron chelator that when administered to the human prevents a further or additional increase in urinary catalytic iron content compared to the catalytic iron content measured in urine prior to treatment or during treatment with the iron chelator. Catalytic iron can be measured in urine of humans undergoing treatment with iron chelator before, during or after the administration of the iron chelator.

In one embodiment, the iron chelator is administered in a single dose. In another embodiment, the iron chelator is administered in multiple doses. In a preferred embodiment, the iron chelator is administered orally at a dose in a range between about 20 mg/kg body of the human and about 150 mg/kg body weight of the human. In a particularly preferred embodiment, the iron chelator is administered three times a day at a dose in a range of between about 20 mg/kg body of the human and about 150 mg/kg body weight of the human for at least 2-6 months.

Another aspect of the invention relates to measuring protein content in urine of a human afflicted with a progressive kidney disease undergoing treatment of the kidney disease with an iron chelator. A measure of protein content can be total protein in the mine (also referred to herein as total protein content). Protein content in urine of the human afflicted with the kidney disease can be measured at one or more time points before, during or after the administration of the iron chelator.

Typically, protein content in urine is determined using routine assays well-known to one of skill in the art. Suitable methods include dipstick, immunoprecipitation, turbidimetric (e.g., sulphosaliclic acid, tricholoracetic, benzethonium chloride) assays, dye-binding (e.g., Coomassie Blue, Ponceau) assays, Biuret (e.g., precipitation with Tsuchiya reagent) assays and Folin-Lowry assays. ("Oxford Textbook of Clinical Nephrology" eds. Davison, AM., et al., 2nd edition, Oxford University Press, New York, NY (1998); "Primer on Kidney Diseases" ed., Greenberg, A., 2nd edition, Academic Press, New York, NY (1998).

In another embodiment, creatinine, blood urea nitrogen, or both are measured in a blood sample obtained from the human at one or more time points before, during or after the administration of the iron chelator to a human afflicted with a kidney disease. The blood sample can be an arterial blood sample or venous blood sample. The blood sample can be a serum or plasma blood sample. Methods to obtain blood samples and process the blood sample to determine blood urea nitrogen content and creatinine content are well known to one of skill in the art. (See, for example, Karlinsky, M.L. et al., Kidney Int. 17:293-302 (1980); Tomford, R.C. et al., J. Clin. Invest. 68:655-664 (1981); Baliga, R. et al., Biochem J. 291:901-905 (1993)).

Blood urea nitrogen, urinary protein content or serum creatinine content in a human afflicted with a kidney disease can increase in the human as the kidney disease progresses. Likewise, blood urea nitrogen, total urinary protein content and serum creatinine content in a human afflicted with a kidney disease can decrease or stabilize (e.g., remain the same, essentially constant) when the progression of a kidney disease is halted or prevented by the administration of iron chelators using the methods of the invention.

In a preferred embodiment, the iron chelator is administered to the human with a kidney disease in an amount effective to lower total protein content in urine, blood urea nitrogen or creatinine content in a blood sample of the human to about that of a control level.

In another embodiment, the iron chelator is administered to the human with a kidney disease in an amount effective to prevent any further increase in total protein content in urine, blood urea nitrogen and creatinine content in a blood sample of the human compared to an amount of catalytic iron prior to treatment with the iron chelator (also referred to herein as a pretreatment amount of urinary catalytic iron).

In yet another embodiment, the iron chelator is administered to the human with a kidney disease in an amount effective to prevent an increase in total protein content in urine, blood urea nitrogen and creatinine in a blood sample of the human to a level above a control level.

In particular, the invention relates to a method of treating a kidney disease in a human, comprising administering a dose of an iron chelator (e.g., in a range between about 20 mg iron chelator/kg body weight and about 150 mg iron chelator/kg body weight) to a human having a catalytic iron content in urine exceeding that of a catalytic iron content in a control sample (e.g., above about 15 nmol catalytic iron/mg of a reference protein). The dose which the human is initially treated with is also referred to as an "initial dose."

In one embodiment, a urine sample is obtained from a human before the administration of the iron chelator (also referred to herein as a pretreatment urine sample). In another embodiment, a urine sample is obtained after the administration of the iron chelator. Thus, a urine sample can be obtained from the human before, during or after the human is administered the iron chelator and catalytic iron content measured.

In a preferred embodiment, the iron chelator (e.g., deferiprone) is administered to a human with a catalytic iron content in a urine sample in an amount greater than about 15 nmol/mg of a reference protein (e.g., urinary creatinine) in a dose in a range of between about 20 mg/kg body weight and about 150 mg/kg body weight of the human.

As defined herein, "control sample" means a level (also referred to herein as amount or content) of the parameter of interest (e.g., catalytic iron content in urine, protein content in urine, blood urea nitrogen in a blood sample, creatinine in a blood sample) in a human not afflicted with a kidney disease, matched, as necessary, for variables, such as age, sex, ethnicity and health history, with the human to be treated. A control sample can also be the expected level of the parameter of interest in a human. The "expected level" of the parameter of interest in a human treated by the methods of the invention can be a level normally observed in a human not afflicted with the kidney disease, or can be higher than a human not afflicted with the kidney disease yet below the level in a human with a kidney disease. An expected level can also be any level of the parameter of interest that is below pretreatment levels or lower than a level expected during progression of a kidney disease in a human not undergoing treatment by the methods defined herein. A "target level" of the parameter of interest can be selected for a human afflicted with a kidney disease based on a level of the parameter of interest (e.g., catalytic iron content in the urine) of a human before that human developed a kidney disease, or in comparison to levels observed in a human not exhibiting a kidney disease. In a preferred embodiment, a control sample has a catalytic iron content of about 15 nmol/mg of a reference protein (e.g., creatinine).

After administration of the iron chelator, the catalytic iron content in the human is measured in a first urine sample. A "first urine sample," when referring to methods of treating the progressive kidney disease, refers to a urine sample obtained from the human at any time after the administration of the iron chelator. The first urine sample can be obtained, for example, days, weeks, months, or years after the administration of the iron chelator. The catalytic iron content in a urine sample (e.g., first urine sample) is compared to the catalytic iron content in the control sample. The catalytic iron content in the first urine sample can also be compared to the catalytic iron content in a urine sample obtained from the human prior to or after administering the initial dose of the iron chelator.

The method can further include administering a subsequent dose of the iron chelator to the human. The amount of the subsequent dose administered is determined by comparing the catalytic iron in the first urine sample with the catalytic iron content in the control sample.

The method can further include obtaining as least one subsequent urine sample from the human. A "subsequent urine sample" is any urine sample obtained from the human at any time after the first urine sample. One or more subsequent urine samples can be obtained from the human. More than one subsequent urine sample can be obtained in a day or over several days, months or years.

The catalytic iron content in the subsequent urine sample of the human is compared with the catalytic iron content in the first urine sample, the control sample, or both. The dose of the iron chelator administered to the human can be adjusted (e.g., increased) until the catalytic iron content in the urine of the human is about that of the control sample or until the catalytic iron content in the subsequent urine sample is essentially constant. The adjusted dose is also referred to as a "subsequent dose." For example, if the human was administered about 75 mg/kg body weight dose of iron chelator and the catalytic iron content in a subsequent urine sample was about 35 nmol/mg of a reference protein compared to about 55 nmol/mg of a reference sample in the urine sample or first urine sample, the subsequent dose of iron chelator could be adjusted to below about 75 mg/kg body weight.

In another embodiment of the invention, the subsequent dose of the iron chelator is administered until the catalytic iron in a subsequent urine sample is about that of the control or essentially constant. "Essentially constant" (also referred to herein as "an essentially constant value" or stabilized) refers to a catalytic iron content in the urine that remains about the same value over time (e.g., days, weeks, months, or years).

In a preferred embodiment, when the catalytic iron content in urine is about that of a control sample or essentially constant, the dose of iron chelator in at least two subsequent doses differs by about 10 mg/kg of body weight of the human. After a 10mg/kg increment adjustment, the catalytic iron content in one or more subsequent urine samples can be measured, compared to the control or an essentially constant value and the dose of iron chelator adjusted (e.g., increased) if the catalytic iron content in the subsequent urine sample is above a control sample or the essentially constant value. For example, if the catalytic iron content in a subsequent urine sample of a human on a maintenance dose of the iron chelator is greater than the control sample (e.g., about 15 nmol/mg of a reference protein) the dose of iron chelator can be increased to decrease the catalytic iron in the urine of the human afflicted with a kidney disease. Similarly, the dose of iron chelator can be decreased if the catalytic iron content in the subsequent urine sample remains, for example, at a control content or essentially constant.

In yet another embodiment, urinary protein content (e.g., total protein content) in a urine sample (e.g., before or after treatment with the iron chelator) and at least one subsequent urine sample is measured. The urinary protein content in the subsequent urine sample is determined and compared to the protein content in a urine sample obtained before or after iron chelator treatment, a control sample or both. Urinary protein content in a subsequent urine sample can be compared to a control sample to determine the progress of treatment, the need for further treatment (e.g., number or duration of doses) or the need to adjust the dose (e.g., mg/kg body weight) of iron chelator being administered to the human.

"Adjusting the dose" of the iron chelator (also referred to herein as a subsequent dose of iron chelator) refers to any change or alteration (e.g., increase or decrease) in the amount of iron chelator in the initial dose. A change in the amount of iron chelator can be, for example, an increase or decrease in the frequency (e.g., times per day, number of days, number of months, number of years) of administration of the iron chelator. Additionally, or alternatively, a change in the amount of iron chelator can be an increase or decrease in the dose (e.g., milligrams of iron chelator per kilogram body weight of the human) of iron chelator administered to the human.

In another embodiment of the invention, creatinine content or blood urea nitrogen content in a blood sample obtained from the human is measured at one or more points before, during or after administration of the iron chelator to a human afflicted with a kidney disease, and who has a catalytic iron content in urine greater than a control sample.

Additionally indices of reducing the severity of the progressive kidney disease can be a decrease in the expected rate at which, for example, blood urea nitrogen, serum creatinine increases, glomerular filtration rate declines, or the onset of end-stage renal disease is delayed. A decline in glomerular filtration rate can be assessed, for example, by an increase in total urinary protein.

A blood sample obtained from the human before administration of an iron chelator is referred to as a "pretreatment blood sample." A blood sample obtained from the human after or during the administration of the iron chelator is also referred to as a "subsequent blood sample." The blood urea nitrogen content and creatinine content in at least one subsequent blood sample is compared with the blood urea nitrogen content and creatinine content in at least one pretreatment blood sample or a control sample. Similarly, the blood urea nitrogen content and creatinine content is compared in at least one subsequent blood sample obtained from the human at different times following the administration and during the course of iron chelator treatment.

The dose of iron chelator can be adjusted depending upon protein content in urine, blood urea nitrogen, creatinine content in the subsequent blood sample, or any combination thereof. For example, a decline (also referred to herein as a decrease) in urinary protein, blood urea nitrogen or creatinine content in a subsequent urine or blood sample compared to a pretreatment sample or a control sample is indicative that the kidney disease is being treated by the iron chelator and the dose of iron chelator could be decreased.

In another embodiment of the invention, a kidney disease in a human is diagnosed. Catalytic iron content is measured in a urine sample obtained from the human and compared with catalytic iron content in a control sample. In this embodiment, catalytic iron content in the urine sample obtained from the human above the catalytic iron content in the control sample is indicative of kidney disease.

In another embodiment, the invention relates to a method of reducing the severity of a progressive kidney disease in a human having a catalytic iron content in a urine sample greater than about 15 nmol/mg of a reference protein. The human afflicted with the progressive kidney disease (e.g., diabetic nephropathy, primary glomerulonephritis, secondary glomerulonephritis) is administered an iron chelator. The iron chelator decreases catalytic iron content in urine obtained from the human thereby reducing the severity of the progressive kidney disease.

The phrase "reducing the severity" (also referred to herein as a reduction in the severity) when referring to a progressive kidney disease means any diminution, amelioration or decrease in progressive damage to the kidney that compromises the function of the kidney. Well- recognized indices to assess function of the kidney can be employed to determine a reduction in the severity of the kidney disease. These indices can include, for example, a reduction in protein content in urine, a reduction in blood urea nitrogen, a reduction in serum or plasma creatinine, a decline in glomerular filtration rate, a delay in the onset of end-stage renal disease, or any combination thereof, compared to a sample obtained from the human prior to administering the iron chelator, or a control sample.

A reduction in the severity of the kidney disease can result, for example, from an amelioration of a primary pathology of the kidney (e.g., injury to the glomerulus or tubule) or another organ (e.g., pancreas) which had adversely affected the ability of the kidney to perform biological functions (e.g., retain protein). Thus, a reduction in the severity of the kidney disease in the human can be the direct or indirect effect of a reduction of the kidney disease.

Another embodiment of the invention is a method of determining the progression of a kidney disease in a human comprising measuring catalytic iron in one or more urine samples obtained from the human. An increase in the catalytic iron content in a urine sample from the human over time (e.g., weeks, months, years) is indicative of the progression of the kidney disease. The catalytic iron content in urine can be measured relative to a reference protein (e.g., creatinine).

"Progression of a kidney disease" refers to an augmentation or increase in the disease of the kidney. Progression of a kidney disease can include, for example, continued or additional damage to a segment of the kidney (e.g., nephron, tubule, interstitium) that was not damaged when the human was initially afflicted or diagnosed with the kidney disease. For example, the human could have no glomerular damage or damage only to the glomerulus resulting in a kidney disease which, over time, leads to increased damage only to the glomerulus or results in tubular damage as the kidney disease progresses. As the kidney disease progresses in the human, the catalytic iron content in urine can increase above a level prior to additional damage to the kidney or in comparison to a human not affected with a progressive kidney disease. Catalytic iron content in urine obtained from the human with progressive kidney disease can be determined and compared to urinary catalytic iron content in another, different human with various stages of progressive kidney disease to discern the progression of the kidney disease.

Progression of the kidney disease can also be monitored, for example, by measuring protein content, in addition to catalytic iron content, in urine of the human afflicted with the kidney disease. As the kidney disease progresses, the protein content in the urine can increase. Additionally, progression of the kidney disease in the human can be assessed by measuring the blood urea nitrogen content or creatinine content in a blood sample obtained from the human over time. An increase in the blood urea nitrogen content or creatinine content over time in the human is also indicative of the progression of the kidney disease in the human. Similarly, a decline in glomerular filtration as assessed, for example, by creatinine clearance as described above can also be used in conjunction with urinary catalytic iron content as indicative of progression of the kidney disease.

In another embodiment, the invention relates to a method of determining the progression of kidney disease in a human comprising measuring catalytic iron content in a first urine sample and in at least one additional urine sample obtained from the human. When determining the progression of a kidney disease, a "first urine sample" refers to an initial sample of urine obtained from the human at anytime prior to determining the progression of the kidney disease. An "additional urine sample" refers to a urine sample obtained from the human at any time after the first urine sample.

The catalytic iron content in the additional urine sample is compared to the catalytic iron content in at least one additional urine sample. An elevation in the catalytic iron in an additional urine sample compared to a first urine sample is indicative of progression of the kidney disease. Likewise, a reduction in the catalytic iron in an additional urine sample compared to the first urine sample is indicative that the kidney disease is not progressing or is being reduced in severity.

The method of determining the progression of kidney disease in a human can further include measuring protein content in the first urine sample and at least one additional urine sample and creatinine content in a first blood sample and at least one additional blood sample obtained from the human afflicted with the kidney disease. A "first blood sample" refers to an initial blood sample obtained from the human at anytime prior to determining the progression of the kidney disease. An "additional blood sample" refers to a blood sample obtained from the human at anytime after the first blood sample.

The protein content in an additional urine sample can be compared to the protein content in the first urine sample and the creatinine content in the additional blood sample can be compared to the first blood sample. An elevation in protein content in the additional urine sample above protein content in the first urine sample and/or an elevation in creatinine content in the additional blood sample above the creatinine content in the first blood sample can be indicative of the progression of the kidney disease in the human. Likewise, a decrease in protein content in the additional urine sample below the protein content in the first urine sample and/or an decrease in creatinine content in the additional blood sample below the creatinine content in the first blood sample is indicative that the kidney disease is not progressing in the human. Similarly, an essentially constant protein content in the additional urine sample can indicate that the kidney disease is not progressing.

Additionally the method of determining the progression of kidney disease in the human can further include comparing blood urea nitrogen in additional blood sample compared to a first blood sample. An elevation in blood urea nitrogen content in the additional blood sample above blood urea nitrogen content in the first blood sample is indicative of the progression of the kidney disease in the human. Similarly, a decrease in blood urea nitrogen content or an essentially constant blood urea nitrogen content in the additional blood sample below blood urea nitrogen content in the first blood sample is indicative the kidney disease is not progressing.

In another embodiment of the invention, the effectiveness of treatment with an iron chelator in a human suffering from a kidney disease is evaluated. Catalytic iron content in at least two urine samples are obtained from the human and compared. "Evaluating the effectiveness" refers to assessing the success of treating the human afflicted with a kidney disease, in particular a progressive kidney disease, and especially a progressive glomerular kidney disease with an iron chelator to the extent that the kidney disease is treated (e.g., that the kidney disease does not progress).

The urine samples obtained from the human to evaluate the effectiveness of treatment with the iron chelator includes a pretreatment urine sample obtained from the human at a pretreatment time and at least one subsequent urine sample obtained from the human at least at one subsequent time after the pretreatment sample. A "pretreatment time" refers to anytime prior to the administration of the iron chelator. A "subsequent time" refers to anytime after the administration of the iron chelator.

A decrease in catalytic iron content in the subsequent urine sample to an amount below a catalytic iron content in the pretreatment urine sample is indicative of the effectiveness of treatment of the kidney disease. Evaluation of the effectiveness of treatment with an iron chelator can further include comparing protein content in the pretreatment urine sample and at least one subsequent urine sample. A decrease in protein content in the subsequent urine sample below protein content in the pretreatment urine sample is indicative of the effectiveness of treatment with an iron chelator in a human suffering from a kidney disease.

In yet another embodiment of the invention, evaluation of the effectiveness of treatment with an iron chelator can further include measuring blood urea nitrogen content and creatinine content in a first blood sample and in a subsequent blood sample obtained from the human. The blood urea nitrogen content and creatinine content in the subsequent blood sample is compared with blood urea nitrogen content and creatinine content in the first blood sample. A decrease in blood urea nitrogen content or creatinine content in the subsequent blood sample below blood urea nitrogen content or creatinine content in the first blood sample is indicative of the effectiveness of treatment with an iron chelator in a human suffering from a kidney disease.

Thus, the effectiveness of treatment with an iron chelator can be evaluated by assessing decreases in urinary catalytic content, urinary protein content, blood urea nitrogen content and creatinine content in a blood sample compared to a pretreatment sample or a control sample.

Another embodiment of the invention relates to a method of slowing the progression of a kidney disease in a human having catalytic iron in urine greater than about 15 nmol/mg of a reference protein, comprising administering an iron chelator to the human. The iron chelator can decrease the catalytic iron content in urine obtained from the urine thereby slowing the progression of the kidney disease in the human.

"Slowing the progression" of a kidney disease refers to reducing the rate or speed at which, or degree to which a kidney disease advances or develops. For example, the progression of a kidney disease can be slowed so that additional glomerular damage or damage to the tubules in a kidney that began with glomerular damage does not occur until a time later than similar damage to the kidney in a human afflicted with the kidney disease who is not administered an iron chelator. Thus, slowing the progression of kidney disease also refers to an increase in the amount of time (e.g., days, months, years) it would take to lead to progressive kidney disease and, particularly progressive glomerular kidney disease or, ultimately, end-stage renal disease in a human who was not administered the iron chelator.

A decrease in catalytic iron content after administration of the iron chelator to the human below a catalytic iron content measured before administering the iron chelator can be used as an indice to assess whether the progression of the kidney disease has been slowed. Additionally, or alternatively, other parameters which is indicative of a slowing of the progression of kidney in a human administered an iron chelator can include, for example, decrease in the urinary protein content, blood urea nitrogen or creatinine in a blood sample obtained from the human during or after administration of the iron chelator compared to urinary protein content, blood urea nitrogen or creatinine in a blood sample obtained from the human before administering the iron chelator, or to a level about that of a control sample.

In yet another embodiment, the invention relates to a method of identifying a human suffering from a progressive kidney disease who will benefit from treatment of the progressive kidney disease with an iron chelator. The phrase "who will benefit" refers to a human who has a catalytic iron content in urine greater than about 15 nmol/mg of a reference protein or who can be treated with an iron chelator to, slow the progression of the kidney disease, reduce the severity of the kidney disease or halt the progression of the kidney disease in the human, and particularly, progressive glomerular kidney disease in the human.

To identify a human who will benefit from treatment of a progressive kidney disease, catalytic iron content is measured in a urine sample obtained from the human. A catalytic iron content in the urine sample greater than about 15 nmol/mg of a reference protein identifies a human suffering from a progressive kidney disease who will, or may, benefit from treatment of the progressive kidney disease with the iron chelator.

In still another embodiment, the invention relates to a method of halting the progression of a kidney disease in a human. "Halting" when referring to the progression of a kidney disease in a human refers to stopping, either temporarily or permanently, the progression of the kidney disease in the human. The progression of the kidney disease can be halted, for example, by preventing or diminishing any further damage to any segment of the kidney (e.g., glomerulus, tubules, interstitum) damaged in the human with the progressive kidney disease. Thus, halting of the progression of a kidney disease in a human can be a diminution in glomerular damage or prevention of damage to a segment of the kidney not otherwise damaged in the human with the progressive kidney disease (e.g., preventing tubular damage in a human with glomerular kidney damage).

To halt the progression of a kidney disease in a human, catalytic iron content and protein content is measured in a pretreatment urine sample obtained from the human. An iron chelator is administered to a human having a catalytic iron content in the pretreatment urine sample in an amount greater than about 15 nmol/mg of a reference protein. The iron chelator can be administered in a dose of about 20 mg/kg body weight to about 150 mg/kg body weight of the human. In a preferred embodiment, the iron chelator is deferiprone.

The catalytic iron content and protein content in the human is measured in at least one subsequent urine sample obtained from the human during or after the administration of the iron chelator. The catalytic iron content and protein content in the subsequent urine sample of the human is compared with the catalytic iron content and protein content in a urine sample (e.g., pretreatment urine sample, first urine sample). In one embodiment, the dose of iron chelator administered to the human is adjusted (e.g., increased or decreased compared to the dose initially administered to the human) until the catalytic iron content in a subsequent urine sample of the human is below the control (e.g., 15 nmol/mg of a reference protein). When the protein content in a subsequent urine sample is about twenty five percent (25%) below the protein content in a control sample, the human is maintained on a dose of iron chelator until no further reduction in urinary protein occurs or until the urinary protein is essentially constant (e.g., for 3-4 months). The dose of iron chelator is then reduced by a dose of about 10 mg/kg body weight.

Urinary catalytic iron content is measured in at least one subsequent urine sample following the 10 mg/kg reduction in dose of the iron chelator. The dose of iron chelator can be further reduced by an additional increment for 10 mg/kg body weight as long as the catalytic iron remains essentially constant or at a control level. Urinary catalytic iron content, urinary protein, blood urea nitrogen and creatinine in a blood sample are monitored following each reduction in the dose of iron chelator. If the urinary catalytic iron, total urinary protein, blood urea nitrogen or creatinine in a blood sample increases, the dose of iron chelator can be increased by an increment of 10 mg/kg of body weight of the human. In a preferred embodiment, about every three months the dose of iron chelator is reduced by about 10 mg/kg of body weight. The human can be maintained on a dose of iron chelator about 10 mg/kg body weight higher than the last dose which results in an essentially constant amount of urinary catalytic iron, total urinary protein, blood urea nitrogen or serum creatinine (also referred to herein as a "maintenance dose" of iron chelator).

Catalytic iron content in urine, urinary protein content, blood urea nitrogen or creatinine in blood can be measured while the human is on a maintenance dose of iron chelator and the dose of iron chelator adjusted to, for example, achieve a urinary protein content about that of a control sample. Similarly, if the catalytic iron content in a subsequent urine sample obtained from a human on a maintenance dose of iron chelator is greater than about 15 nmol/mg of a reference protein, the dose of iron chelator can be increased (e.g., to about 20-150 mg/kg of body weight).

The dose of iron chelator can be further increased if the catalytic iron content in the urine sample obtained from the human continues to rise following an adjustment in the dose of iron chelator above the maintenance dose. Similarly, the dose of iron chelator can be decreased if the catalytic iron content in the urine sample obtained from the human decreases following an increase in the dose of iron chelator compared to the maintenance dose. The need to further adjust the dose of iron chelator can be determined by monitoring catalytic iron content in urine, protein content in urine, blood urea nitrogen, creatinine in a blood sample, or any combination thereof.

The invention further relates to the use of iron chelators to diagnose and treat humans exhibiting microalbuminuria. In a preferred embodiment, the iron chelator is deferiprone.

The term "microalbuminuria" refers to any disease, disorder, ailment or state of health where urinary albumin is excreted at a rate of about 20-200 $\mu$g/minute or about 30-300 mg/24 hours. (see, for example, Abbott, K.C., et al., Arch. Internal Med. 154:146-153 (1994)). Methods to detect and diagnose microalbuminuria are well known to one of skill in the art and include radioimmunoassays, immunoassays with latex bodies, fluoroimmunoassays, enzyme immunoassays, agglutination inhibition, immunoturbidimetry, immunonephelometry and radial immunodiffusion assays. (Keen, H. et al., Lancet 2:913-916 (1968); Silver, A. et al., Clin. Chem 32: 1303-1306 (1986); Close, C. et al., Diabet. Med. 4:491-492 (1987); Harmoinen, A. et al., Clin. Chim. Acta 166:85-89 (1987); Marre, M. et al., Clin.. Chem. 33: 209-213 (1987); McCormik, C.P. et al., Ann. Clin. Lab Sci. 19:944-951 (1989); Cambiaso, C.L. et al., Clin. Chem. 34: 416-418 (1988); Niwa, T. et al., Clin. Chim. Acta 186:391-396 (1990). The levels of albumin or total protein in urine before and after treatment with the iron chelator can be determined and the frequency or amount of doses required to alleviate microalbuminuria can be adjusted as needed for each individual undergoing treatment. Since microalbuminuria is associated with diabetic nephropathy in diabetic individuals (Mattock, M.B., et al., Diabetes 41:736-741 (1992); Neil, A., et al., Diabetes Care 16:996-1003 (1993); Abbott, K.C., et al., Arch. Intern. Med. 154:146-153 (1994)) it is expected that the methods of the invention can be employed to halt the progression of microalbuminuria and, thus, prevent development of diabetic nephropathy.

The amount effective to alleviate microalbuminuria can be an amount of the iron chelator that restores the levels of albumin in urine or prevents a further increase in albumin levels in the urine. In a preferred embodiment, the levels of albumin in urine are restored to levels of about that in a control individual. The term "restore" refers to returning the albumin levels in urine to the levels observed before the individual developed microalbuminuria or to levels of about that observed in a control individual. The administration of iron chelators to humans afflicted with microalbuminuria can be used to halt the progression of the microalbuminuria. In one embodiment, progression of micoralbuminuria is halted by administering to the human an iron chelator (e.g., about 20 mg/kg body weight to about 150 mg/kg body weight).

In another embodiment, the invention relates to a method of halting the progression of microalbuminuria in a human, comprising measuring catalytic iron content in a pretreatment urine sample obtained from the human and administering a dose of an iron chelator to a human having a catalytic iron content in the pretreatment urine sample above a catalytic iron content in a control sample. The catalytic iron content in at least one subsequent urine sample obtained from the human is measured during or after the administration of the iron chelator and compared with the catalytic iron content in the pretreatment urine sample, the control sample, or both. The dose of the iron chelator administered to the human is adjusted until the catalytic iron content in the urine of the human is about that of the control sample.

A "control" level, when referring to the treatment of humans exhibiting microalbuminuria, is defined as described above for humans afflicted with kidney diseases except the control level refers to a level of urinary albumin, rather than a level of total protein as described in humans afflicted with kidney disease.

Microalbuminuria can progress and ultimately lead to diabetic nephropathy, a progressive kidney disease (Mattock, M.B., et al., Diabetes 41:736-741 (1992); Neil, A., et al., Diabetes Care 16:996-1003 (1993); Abbott, K.C., et al., Arch. Intern. Med. 154:146-153 (1994)). Thus, another aspect of the invention relates to a method of halting the progression of microalbuminuria in a human comprising measuring catalytic iron content in a pretreatment urine sample obtained from the human and administering a dose of deferiprone to a human having a catalytic iron content in the pretreatment urine sample in an amount greater than about 15 nmol/mg of a reference protein in a dose of about 20 mg/kg body weight to about 150 mg/kg body weight of the human. The catalytic iron content is measured in at least one subsequent urine sample obtained from the human during or after the administration of deferiprone. The catalytic iron content in the subsequent urine sample of the human is compared with the catalytic iron content in the pretreatment urine sample and the dose of deferiprone administered to the human adjusted until the catalytic iron content in a subsequent urine sample of the human is below about 15 nmol/mg of a reference protein.

The methods of the present invention can be accomplished by the administration of the iron chelator iron by enteral or parenteral means. Specifically, the route of administration is by oral ingestion (e.g., tablet, capsule form). Other routes of administration as also encompassed by the present invention including intramuscular, intravenous, intraarterial, intraperitoneal, or subcutaneous routes, and nasal administration. Suppositories or transdermal patches can also be employed.

The iron chelators can be used alone or in any combination when administered to the humans. For example, deferiprone can be coadministered with another iron chelator such as deferoxiamine to treat a kidney disease (e.g., diabetic nephropathy, primary glomerulonephritis, secondary glomerulonephritis, microalbuminuria). It is also envisioned that one or more iron chelators can be coadministered with other therapeutics (e.g., steroids) to, for example, treat kidney diseases, halt the progression of a kidney disease, reduce the severity of a kidney disease Coadministration is meant to include simultaneous or sequential administration of two or more iron chelators. It is also envisioned that multiple routes of administration (e.g., intramuscular, oral, transdermal) can be used to administer one or more iron chelators.

The iron chelators can be administered alone or as admixtures with conventional excipients, for example, pharmaceutically, or physiologically, acceptable organic, or inorganic carrier substances suitable for enteral or parenteral application which do not deleteriously react with the iron chelator. Suitable pharmaceutically acceptable carriers include water, salt solutions (such as Ringer's solution), alcohols, oils, gelatins and carbohydrates such as lactose, amylose or starch, fatty acid esters, hydroxymethycellulose, and polyvinyl pyrolidine. Such preparations can be sterilized and, if desired, mixed with auxillary agents such as lubricants, preservatives, stabilizers, wetting agents, emulsifiers, salts for influencing osmotic pressure, buffers, coloring, and/or aromatic substances and the like which do not deleteriously react with the iron chelator.

When parenteral application is needed or desired, particularly suitable admixtures for the iron chelator are injectable, sterile solutions, preferably oily or aqueous solutions, as well as suspensions, emulsions, or implants, including suppositories. In particular, carriers for parenteral administration include aqueous solutions of dextrose, saline, pure water, ethanol, glycerol, propylene glycol, peanut oil, sesame oil, polyoxyethylene-block polymers, and the like. Ampules are convenient unit dosages. The iron chelators can also be administered via transdermal pumps or patches. Pharmaceutical admixtures suitable for use in the present invention are well-known to those of skill in the art and are described, for example, in Pharmaceutical Sciences (17th Ed., Mack Pub. Co., Easton, PA) and WO 96/05309 the teachings of both of which are hereby incorporated by reference.

The dosage and frequency (single or multiple doses) of iron chelators administered to a human can vary depending upon a variety of factors, including the size, age, sex, health, body weight, body mass index, and diet of the human; nature and extent of symptoms of the kidney disease being treated (e.g., diabetic nephropathy, microalbuminuria), kind of concurrent treatment (e.g., steroids), complications from the kidney disease, microalbuminuria, or other health-related problems. In a preferred embodiment, humans with a kidney disease are treated three times a day with a dose of iron chelator (e.g., deferiprone in 500 mg capsules) at about 30 mg/kg to about 75 mg/kg body weight per day for about 2-6 months. Other therapeutic regimens or agents can be used in conjunction with the iron chelator treatment methods of the present invention. For example, the administration of the iron chelator can be accompanied by steroid administration. Adjustment and manipulation of established dosages (e.g., frequency and duration) are well within the ability of those skilled in the art.

The present invention is further illustrated by the following examples, which are not intended to be limiting in any way.

EXEMPLIFICATION

**EXAMPLE 1: URINARY CATALYTIC IRON IN PATIENTS WITH KIDNEY DISEASE**

Selection of Patients with Kidney Disease

Human patients (n=70) were used in these studies. Controls were healthy human patients with no history of kidney disease, hypertension, diabetes or other diseases that affect the kidney. Males and females were used in the studies. Ages of the human patients ranged from 4 years of age to 70 years of age. Ethnicity of the patients included Caucasians, African-American and Hispanics.

Kidney disease was detected in patients by the presence of an abnormal amount of protein in the urine and/or an abnormal renal function. An abnormal amount of protein in urine is an amount of total protein greater than about 0.15g/24h measured by an adaptation of pyrogallol red-molydate method described by Fujita, Y., et al., Bunseki Kgaku 32:379-386 (1983). Many patients, as part of their routine work up, had received kidney biopsies providing diagnosis of the nature of the kidney disease.

Glomerular filtration rate was assessed in humans by measuring serum creatinine (normal values 0.96 mg/dl for women, and 1.16 mg/dl for men - values above 1.4 mg/dl considered abnormal), creatinine clearance or both. Creatinine clearance was calculated by the standard formula Cr = Ucr V/PCr where Cr = clearance of creatinine (ml/min); Ucr = urine creatinine (mg/dl), V = volume of urine, and PCr = plasma creatinine (mg/dl).

Patients with kidney disease included the following groups of patients:

Glomerulonephritis. This diagnosis was based on patients with abnormally high amount of urinary protein (greater than about 150 mg/24 hours) including patients with nephrotic syndrome (greater than about 3.5 gm protein) .The diagnosis of glomerulonephritis was made on histological examination of the kidney, which included light microscopy, immunofluorescence, and electron microscopy using routine methods. Diagnoses included both primary causes such as membranous nephropathy, focal segmental sclerosis, IgA nephropathy, membranoproliferative glomerulonephritis, and crescentic glomerulonephritis as well as secondary causes such as, systemic lupus erythematosis, hemolytic uremic syndrome or Henoch-Schonlein purpura.

Microalbuminuria Patients with diabetes who had microalbuminuria had urinary albumin in a range between about 30 mg to about 300 mg for 24 hours as measured by a method which uses the rate of increase in light scattered from particles suspended in solution as a result of complexes formed during an antigen-antibody reaction or by the dipstick method.

Diabetic Nephropathy. Patients with diabetes who had diabetic nephropathy with overt proteinuria (greater than about 150 mg/24 hours) in 24-hour sample . The diagnosis of diabetic nephropathy was based on presence of overt proteinuria, history of diabetes, and presence of retinopathy on clinical examination.

Ischemic nephropathy was based on clinical and radiological findings.

A urine sample was obtained from humans with and without (control) kidney disease and assessed for catalytic iron content and creatinine content using established methods as described below.

Urinary Catalytic Iron and Creatinine in Humans With and Without Kidney Disease

Catalytic iron content was measured in urine using a well established bleomycin assay (Gutteridge J.M.C., et al., Biochem J 199:263-265 (1981), the teachings of which are incorporated herein in their entirety).

*In vivo* iron is primarily bound to heme or non-heme proteins and does not directly catalyze the generation of hydroxyl radicals or a similar oxidant (Gutteridge J.M.C., et al., Biochem J 199:263-265 (1981)). In the catalytic iron assay used in these studies, the antibiotic bleomycin detects iron complexes capable of catalyzing free radical reactions. Bleomycin, in the presence of an iron salt and a suitable reducing agent, binds to and degrades DNA with the formation of a product that reacts with thiobarbituric acid to form a chromogen. The binding of the bleomycin-iron complex to the DNA makes the reaction site specific and antioxidants rarely interfere. Thus, the iron detected by this method was a measure of iron available from the sample to bleomycin. The assay conditions prevent interference from iron containing proteins. Iron bound to transferrin, lactoferrin, ferritin or iron containing enzymes is not detected in the bleomycin assay (Gutteridge J.M.C., et al., Biochem J 199:263-26 (1981)).

Specifically, the assay was performed as follows:

*Reagent preparation*

1. Chelex-treated pyrogen free $H_2O$ (Chelex $H_2O$)

Add 5 g chelex 100 resin (Bio-Rad, Cat#: 142-2842: 200-400 mesh, sodium form) to 100 ml pyrogen free water (Baxter) and shake well. Stand overnight at 4˚C and filter off the Chelex with bottle filter system (0.22 $\mu$m filter). After Chelex treatment of all solutions used in the assay, the Chelex must be carefully removed to avoid contamination by resin.

2. DNA (1mg/ml)

Add 50 mg DNA (Type I: calf thymus, Sigma D-1501) to 50 ml Chelex $H_2O$ and allow to stand at 4˚C overnight to effect solution. Treat this solution with 15 mg Chelex 100 resin overnight at 4˚C and centrifuge at 3000 rpm for 30 min to remove Chelex. If there are still some Chelex particles in the solution, centrifuge it again at 3000 rpm about 15 min. The reconstituted DNA is suitable for use in the assay for about 7 days after mixing when stored at 4˚C.

3. Bleomycin sulfate (1 U/ml)

15 U bleomycin sulfate (Bleoxane, Nippon Kayaku Co. Ltd, Tokyo, Japan) in 15 ml Chelex $H_2O$. The reconstituted bleomycin is suitable for use in the assay for about 1 month after preparation.

4. Magnesium chloride (50 mM)

Add 0.1 g $MgCl_2 \cdot 6H_2O$ (FW. 203.3) in 10 ml Chelex $H_2O$. Treat the solution with 3 g Chelex 100 resin and centrifuge at 3000 rpm for 10 min to remove Chelex.

5. Hydrochloric acid (40 mM)

33.2 $\mu$l HCl (Ultrapure, Baker 6900-05) in 10 ml Chelex $H_2O$.

6. NaOH (40 mM)

16 mg NaOH in 10 ml Chelex $H_2O$.

7. Ascorbic acid (8 mM)

70 mg ascorbic acid (FW. 176.1) in 1 ml Chelex H2O. Treat the solution with 50 mg Chelex 100 resin and centrifuge at 3000 rpm for 30 min to remove Chelex. Dilute 1:50 with Chelex H2O before use. Prepare immediately prior to use in the assay.

8. EDTA (0.1 M)

0.37 g EDTA in 10 ml Chelex $H_2O$.

9. Thiobarbituric acid (TBA) (1% w/v in 50 mM NaOH)

1 g TBA in 100 ml of 50 mM NaOH (NaOH prepared in Chelex $H_2O$).

10. HCl (25% v/v)

25 ml HCl (Ultrapure, Baker Catalog #6900-05) in 100 ml Chelex $H_2O$.

11. Standard Iron

16 mg $FeCl_3$ (FW. 162.2) in 10 ml Chelex $H_2O$. Dilute 1:100 for stock solution (100 nmol/ml), then dilute 1:1 to make the standard concentrations as 50. 25, 12.5, 6.25, and 3.125 nmol/ml and Chelex $H_2O$. Prepare standards immediately prior to use in the assay.

*Procedure*

1. To remove particulates, urine samples were centrifuged at about 3000 rpm for about 10 min and the resulting supernatant used in the bleomycin assay.

2. Place the following in a plastic tube in the following order:

> 0.5 ml DNA
> 0.05 ml bleomycin sulfate (for blanks add Chelex $H_2O$)
> 0.1 ml $MgCl_2$
> 0.1 ml standard Fe solution or urine samples
> 0.1 ml ascorbic acid

3. Mix well before and after the addition of ascorbic acid. For each sample or standard prepare a parallel blank. The sample and standard blanks are identical to their test reaction mixtures except that bleomycin is omitted

4. Adjust pH of all standards, samples and their blanks to about pH 7.2-7.8 using 40 mM HCl or 40 mM NaOH.

5. Incubate all tubes in temperature controlled shaker at 100 rpm, about 37˚C for about 2 h.

6. Stop the reaction by addition of 0.1 ml EDTA.

7. Add 1 ml TBA and 1 ml 25% HCl and mix well.

8. Transfer the mixture into new or acid-washed glass tube and heat at about 100˚C for about 15 min.

9. Transfer the solution in to 4.5 ml cuvette after cooling and read absorbency at 532 nm against the corresponding blank (without bleomycin).

10. Calculate the amounts of bleomycin-detectable iron content in test samples from the standard curve obtained in each experiment. The absorbency of the zero standard (contains only Chelex $H_2O$) reflecting iron contamination in the reagents is subtracted from all test samples.

11. Urine bleomycin-detectable iron is expressed as nmol/mg creatinine. Creatinine in the urine sample is measured using a creatinine assay kit (Sigma Chemical Co., St. Louis, MO 555-A).

Statistical Analysis

Data were expressed as the mean ± SEM of nmoles catalytic iron/mg creatinine. Significant differences between group means was determined using an unpaired t-test. The patient data was compared to controls using a paired t-test and p-value of < 0.05 was considered significant.

Results and Conclusions

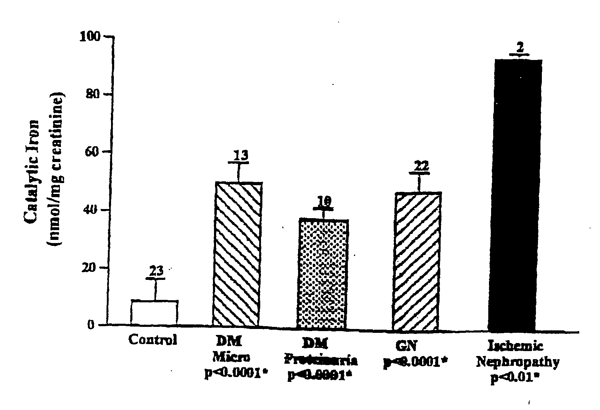

As shown in Figure 1, Table 1 and Table 2, urinary catalytic iron was elevated in patients with a wide variety of kidney diseases compared to patients without kidney disease. Thus, urinary catalytic iron plays a role in kidney disease and is indicative of a kidney disease in a human.

EXAMPLE 2: TREATMENT OF PATIENTS WITH KIDNEY DISEASE WITH AN IRON CHELATOR.

Selection of Patients with Kidnev Disease

The diagnosis of the kidney disease was made by performing a kidney biopsy and included patients with membranoproliferative glomerulonephritis, membranous nephropathy, focal segmental sclerosis, IgA nephropathy, and diabetes as described above.

Iron Chelator Treatment of Patients with Kidney Disease

Patients with kidney disease who had abnormally high urinary protein were treated with the iron chelator deferiprone (1,2-dimethyl-3-hydroxypyrid-4-one) (L1).
Deferiprone was synthesized as previously described capsule (Kontoghiorghes A.J., et al., Inorganica Chimica Acta 136:L11-L12 (1987)) and formulated into capsules containing 500 mg of deferiprone per capsule.

Urinary protein and serum creatinine was measured as described above before the administration of deferiprone. Patients were treated by oral ingestion of deferiprone in a range between about 30 mg and about 75 mg deferiprone per kilogram body weight per day 3 times a day. Urinary protein and serum creatinine was obtained 2-6 months after the administration of the iron chelator. As shown in Table 3, treatment with the iron chelator significantly decreased the amount of total urinary protein and serum creatinine in patients with kidney disease. Total urinary protein and serum creatinine measured before (e.g., pretreatment) and after the administration of deferiprone were compared using a paired t-test and p-value of < 0.05 was considered significant.

These data show that the iron chelator treated the kidney diseases. Therefore, it is believed that the effect of catalytic iron can be significantly reduced or prevented from acting directly or indirectly on the kidney to injure the kidney, thereby causing kidney disease, which if left untreated (no iron chelator) would result in an increase in total urinary protein content and serum creatinine.

### Table 1: Urinary Catalytic Iron In Patients with Progressive Kidney Disease

| Age/Sex/Race | | | Diagnosis | 24 hr prot (mg/24 hrs) | Serum Cr (mg/dl) | CrCl (ml/min) | Catalytic Iron (Nmol/mg creatinine) |
|---|---|---|---|---|---|---|---|
| 20 | F | W | Membranous | 8900 | 1.1 | 120.0 | 33.4 |
| 23 | M | W | Membranous | 4200 | 2.3 | | 223 |
| 50 | F | B | SLE | 5000 | 1.9 | 57.0 | 90.7 |
| 51 | F | B | SLE | 5000 | 1.4 | | 9.1 |
| 64 | M | W | SLE | 3700 | 0.9 | | 11.0 |
| 45 | F | W | SLE | 3700 | 0.6 | | 69.0 |
| 38 | F | B | SLE | 6843 | 4.4 | | 70.0 |
| 38 | F | B | FSGS | 5000 | 2.6 | | 52.7 |
| 7 | M | W | FSGS | 20790 | 2.1 | 30.6 | 48.6 |
| 15 | F | B | FSGS | 2360 | 0.8 | 110.2 | 43.7 |
| 14 | F | B | FSGS | 16350 | 2.3 | 38.6 | 173 |
| 32 | M | B | FSGS | 300 | 2.2 | | 20.1 |
| 40 | F | B | FSGS | 5200 | 1.7 | | 61.6 |
| 40 | M | W | FSGS | | 2.1 | | 28.0 |
| 40 | M | B | FSGS | 15730 | 3.3 | 20.0 | 118.0 |
| 11 | F | W | HSP | 11120 | 0.6 | 139.3 | 26.1 |
| <1 | M | W | HUS | | 0.7 | | 84.2 |
| 16 | M | B | IgA | 830 | 1.6 | 58.4 | 19.1 |
| <1 | M | B | MPGN | 2104 | 0.5 | 75.0 | 66.8 |
| 54 | M | | MPGN | | 3.8 | | 43.0 |
| 40 | M | W | MPGN | 16260 | | | 106.0 |
| 65 | F | W | Creacentic | | 5.9 | | 5.4 |
| Mean | | | | | | | 48 |
| SEM | | | | | | | 7 |
| Number | | | | | | | 22 |
| P | | | | | | | <0.0001 |
| Ischemic Nephropathy | | | | | | | |
| 45 | F | W | Isch Neph | | 1.8 | | 92.0 |
| 80 | F | W | Isch Neph | | 2.0 | | 96.0 |
| Mean | | | | | | | 94.0 |
| SEM | | | | | | | 2.0 |
| Number | | | | | | | 2 |

(continued)

Ischemic Nephropathy

| P | <0.01 |
|---|---|

SLE: Systemic Lupus Erythematosus; FSGS=Focal Segmental Glomerulosclerosis;
HSP=Henoch-Schonlein Purpura; HUS=Hemolytic Uremic Syndrome;
MPGN=Membranoproliferative Glomerulonephritis; Isch Neph=Ischemic Nephropathy SLE, HSP, HUS and
Ischemic Nephropathy are secondary causes of renal disease.
P compared to control value of 8.1 = 1.4, n=23, using unpaired t test
Isch Neph=Ischemic Nephropathy
prot=protein
Cr=creatinine
QrCl=creatinine Clearance

### TABLE 2: Urinary Catalytic Iron In Patients with Diabetic Nephropathy

| Age/Sex/Race | | | 24 hr prot | Serum Cr | CrCl | Catalytic Iron |
|---|---|---|---|---|---|---|
| | | | (mg/24 hrs) | (mg/dl) | (ml/min) | (nmol/mg creatinine) |
| _Microalbuminuria_ | | | | | | |
| 69 | F | W | | 0.8 | | 59.3 |
| 77 | M | W | | 1.4 | | 84.1 |
| 56 | F | W | | 0.7 | | 75.9 |
| 39 | F | B | | 0.6 | | 10.2 |
| 47 | F | W | | 1.0 | | 46.3 |
| 50 | M | B | | 1.3 | 103.0 | 23.0 |
| 56 | M | W | | 2.4 | 46.0 | 21.0 |
| 61 | M | W | | 1.2 | 90.0 | 36.0 |
| 69 | M | W | | 1.1 | 77.0 | 25.0 |
| 73 | M | B | | 1.4 | 54.0 | 58.0 |
| 78 | M | W | | 1.1 | 71.0 | 82.0 |
| 59 | F | B | | 1.2 | 116.7 | 44.0 |
| 30 | M | B | | 1.4 | 68.0 | 86.0 |
| Mean | | | | | | 50.0 |
| SEM | | | | | | 7.0 |
| Number | | | | | | 13 |
| P | | | | | | <0.0001 |
| Diabetic Patients with Overt Proteinerria | | | | | | |
| 61 | M | B | 4800.0 | 5.3 | | 56.2 |
| 56 | M | B | 8000.0 | 3.0 | | 20.0 |
| 67 | F | B | 3816.0 | 3.1 | 25.0 | 42.0 |
| 42 | F | W | 8300.0 | 7.2 | 10.0 | 32.6 |
| 51 | F | B | 11200.0 | 2.8 | 31.0 | 53.8 |
| 32 | M | W | 6899.0 | 6.8 | | 44.5 |
| 61 | M | B | 11363.0 | 5.4 | | 30.2 |
| 73 | M | W | 1064.0 | 3.0 | | 27.3 |
| 71 | F | B | 3200.0 | 1.6 | | 49.0 |
| 79 | F | W | 1350.0 | 3.0 | | 25.0 |
| Mean | | | | | | 38.0 |
| SEM | | | | | | 4.0 |
| Number | | | | | | 10 |

(continued)

Diabetic Patients with Overt Proteinerria

| P | | | | | | | | <0.0001 |
|---|---|---|---|---|---|---|---|---|

P compared to control value of 8.1 ± 1.4, n=23, using unpaired t test
Microalbuminuria is defined as urinary albumin between 30 to 300 mg/24 hrs as measured quantitavely in a 24 hour sample or by a dipstick in a spot urine.
prot=protein
Cr=creatinine
CrCl=creatinine Clearance

**TABLE 3: Urinary protein and creatinine levels in patients with kidney disease before and after treatment with an iron chelator**

| Pt. # | Sex | Age | W t (kg) | Diagnosis* | 24-Hr Urinary Protein grams/24 hrs | | Reduction | | Creatinine mg/dl | |
|---|---|---|---|---|---|---|---|---|---|---|
| | | | | | Before | After | Grams | % | Before After | |
| 1 | F | 52 | 60 | MPGN | 2.6 | 1.2 | 1.4 | 54 | 1.3 | 0.8 |
| 2 | F | 40 | 73 | Membranous | 2.8 | 0.6 | 2.2 | 78 | 1.4 | 0.9 |
| 3 | F | 29 | 42 | Chronic GN | 3.4 | 1.5 | 1.9 | 49 | 1.0 | 0.9 |
| 4 | M | 33 | 74 | Membranous | 1.2 | 0.2 | 1 | 83 | 1.4 | 1.3 |
| 5 | F | 40 | 66 | Membranous | 6.5 | 4 | 2.5 | 38 | 0.8 | 0.9 |
| 6 | M | 28 | | FSGS | 2.8 | 1.6 | 0.7 | 25 | | |
| 7 | F | 18 | 36 | FSGS | 4.2 | 1.6 | 2.6 | 62 | 0.7 | 0.6 |
| 8 | M | 14 | 37 | Mesangial proliferation/Ig A | 6 | 0.05 | 5.95 | 99 | 0.6 | 0.5 |
| 9 | M | 3 | 14 | DPGN | 5.8 | 2.8 | 2 | 34 | | |
| 10 | F | 12 | | MPGN | 4.6 | 2.5 | 2.1 | 44 | 0.4 | 0.5 |
| 11 | M | 32 | 77 | Diabetes | 0.538 | 0.1 | 0.43 | 80 | 1.6 | 1.4 |
| 12 | M | 45 | | Mesangial proliferation/Ig A | 4.5 | 3.8 | 0.7 | 15 | | |
| 13 | M | 35 | 50 | Membrane | 5.3 | 4.8 | 0.5 | 9 | 0.8 | 0.7 |
| 14 | M | 32 | 62 | Membranous FSGS | 4.5 | 4.6 | 0.1 | 2 | 2.1 | 1.5 |
| | | | | **Mean** | **3.91** | **2.13** | **1.70** | **48** | **1.10** | **0.90** |
| | | | | **SD** | **1.78** | **1.67** | **1.49** | **30** | **0.50** | **0.35** |
| | | | | **SEM** | **0.47** | **0.45** | **0.39** | **8** | **0.15** | **0.10** |
| | | | | **N** | **14** | **14** | **14** | **14** | **11** | **11** |
| | | | | | | p<0.001 | p<0.001 | p<0.001 | | |
| | | | | | | | | | | |

DPGN: Diffuse proliferative glomerulonephritis; MPGN: Membranoproliferative glomerulonephritis; FSGS: Focal segmental glomerulosclerosis

EQUIVALENTS

While this invention has been particularly shown and described with references to preferred embodiments thereof, it will be understood by those skilled in the an that various changes in form and details may be made therein without departing from the invention as defined by the appended claims.

**Claims**

1. A method for diagnosing a kidney disease in a human comprising the step of measuring catalytic iron in a urine sample from the human.

2. The method of Claim 1, wherein:

   (a) catalytic iron is measured in relation to a reference protein in the urine sample, and optionally wherein the reference protein is urinary creatinine;
   (b) the urine sample is obtained from a human having ischemic nephropathy, kidney disease consequent to damage to an interstitium of a kidney, a progressive kidney disease selected from the group of consisting of diabetic nephropathy, primary glomerulonephritis and secondary glomerulonephritis, wherein the primary glomerulonephritis is selected from the group consisting of membranous nephropathy, focal segmental glomerulosclerosis, IgA nephropathy, membranoproliferative glomerulosclerosis, IgA nephropathy, membranoproliferative glomerulonephritis and crescentic glomerulonephritis, and wherein the secondary glomerulonephritis is selected from the group consisting of systemic lupus erythematosis, Henoch-Schonlein purpura and hemolytic uremic syndrome;
   (c) the method further comprises comparing catalytic iron content in the urine sample with catalytic iron content in a control sample,
   wherein catalytic iron content in the urine sample above the catalytic iron content in the control sample is indicative of kidney disease;
   (d) the method comprises a method of identifying a human suffering from a progressive kidney disease who will benefit from treatment of the progressive kidney disease with an iron chelator wherein a catalytic iron content in the urine sample greater than about 15 nmol/mg of a reference protein identifies a human suffering from a progressive kidney disease who will benefit from treatment of the progressive kidney disease with the iron chelator; or
   (e) wherein the catalytic iron is measured prior to or after onset of clinical symptoms of the kidney disease.

3. Use of an iron chelator for the manufacture of a medicament for treating a kidney disease in a human.

4. The use of Claim 3 wherein:

   (a) the iron chelator is administered to a human having ischemic nephropathy, a human having the kidney disease consequent to damage to an interstitium of a kidney; a human having a progressive kidney disease selected from the group consisting of diabetic nephropathy, primary glomerulonephritis and secondary glomerulonephritis;
   (b) the iron chelator is selected from the group consisting of deferiprone, deferoxamine, polyanionic amines and substituted polyaza compounds;
   (c) the iron chelator is administered at a dose in a range of between about 20 mg/kg body weight and about 150 mg/kg body weight of the human per day;
   (d) the iron chelator is administered in multiple doses;
   (e) the iron chelator is administered orally;
   (f) the reference protein is urinary creatinine;
   (g) the protein content in a urine sample obtained from the human is measured;
   (h) the catalytic iron content in a urine sample obtained from the human is measured;
   (i) the creatinine in a blood sample obtained from the human is measured;
   (j) the blood urea nitrogen content in a blood sample obtained from the human is measured;
   (k) wherein the administration of the iron chelator reduces deterioration of glomerular filtration rate; or
   (l) wherein administration of the iron chelator reduces one member selected from the group consisting of protein in urine, blood urea nitrogen, serum creatinine and plasma creatinine.

5. The use of Claim 3, wherein

the iron chelator is for administration to a human having catalytic iron content in urine exceeding that of the control sample; and the treatment further comprises

measuring catalytic iron content in a first urine sample taken after administration of the iron chelator; and

comparing the catalytic iron content in the first urine sample with the catalytic iron content in the control sample.

6. The use of Claim 5, wherein further includes the treatment subsequent doses of the iron chelator, the amount of the subsequent dose being determined by comparison of the measured catalytic iron content in the urine sample to the catalytic iron content in the control sample and optionally further including the steps of:

measuring catalytic iron content in a subsequent urine sample;

and

comparing the catalytic iron content in the subsequent urine

sample with the catalytic iron content in the first urine sample,

control sample, or both;

and further optionally wherein the subsequent dose of the iron chelator is administered until the urinary catalytic iron content in a subsequent urine sample is essentially constant;

and optionally either wherein the subsequent dose of the iron chelator is administered until the essentially constant urinary catalytic iron content in the subsequent urine sample is about that of the control sample, or wherein the dose of iron chelator administered in at least two subsequent doses differs about 10 mg/kg of body weight of the human.

7. The use of Claim 5, wherein

(a) creatinine in a blood sample obtained from the human is measured;

(b) blood urea nitrogen content in a blood sample obtained from the human is measured;

(c) the iron chelator is selected from the group consisting of deferiprone, deferoxamine, polyanionic amines and substituted polyaza compounds;

(d) the iron chelator is administered at a dose in a range of between about 20 mg/kg body weight and about 150 mg/kg body of the human per day;

(e) the urine sample is obtained from a human having a progressive kidney disease selected from the group consisting of diabetic nephropathy, primary glomerulonephritis and secondary glomerulonephritis;

(f) the urine sample is obtained from a human having a progressive glomerular kidney disease;

(g) the catalytic iron content is measured in relation to a reference protein, and optionally the reference protein is urinary creatinine;

(h) administration of the iron chelator essentially halts progression of the kidney disease in the human;

(i) administration of the iron chelator reduces the severity of a progressive kidney disease in a human; or

(j) the use is in treating microalbuminuria and optionally further including the step of administering a subsequent dose of the iron chelator, the amount of the subsequent dose being determined by the comparison of the measured catalytic iron content in the urine sample to the catalytic iron content in the control sample, and/or wherein administration of the iron chelator essentially halts progression of microalbuminuria in the human.

8. A method of evaluating the effectiveness of administering an iron chelator to treat a human suffering from a progressive kidney disease, comprising the step of comparing catalytic iron content of a first urine sample obtained from the human with catalytic iron content of a subsequent urine sample, the subsequent urine sample having been obtained after obtaining the first urine sample and after administration of the iron chelator, whereby the comparison indicates the effectiveness of the administration of the iron chelator to alleviate progression of the kidney disease.

**Patentansprüche**

1. Verfahren zum Diagnostizieren einer Nierenerkrankung in einem Menschen mit dem Schritt der Messung von katalytischem Eisen in einer von dem Menschen stammenden Urinprobe..

2. Verfahren nach Anspruch 1, in welchem:

(a) das katalytische Eisen in Bezug auf ein Referenzprotein in der Urinprobe gemessen wird und das Referenzprotein wahlweise das Kreatinin im Urin ist;

(b) die Urinprobe erhalten wird von einem Menschen mit ischämischer Nephropathie, einer Nierenerkrankung in Folge eines Schadens an einem Niereninterstitium, einer progressiven Nierenerkrankung, ausgewählt aus der Gruppe diabetische Nephropathie, primäre Glomerulonephritis und sekundäre Glomerulonephritis, wobei die primäre Glomerulonephritis ausgewählt wird aus der Gruppe membranöse Nephropathie, fokal-segmentale Glomerulosklerose, IgA-Nephropathie, membranoproliferative Glomerulosklerose, IgA-Nephropathie, membranoproliferative Glomerulonephritis und halbmondförmige Glomerulonephritis und wobei die sekundäre Glomerulonephritis ausgewählt wird aus der Gruppe systemischer Lupus erythematodes, Purpura Schoenlein-Henoch und hämolytisch-urämisches Syndrom;

(c) das Verfahren ferner einen Vergleich des Gehalts an katalytischem Eisen in der Urinprobe mit dem Gehalt an katalytischem Eisen in einer Kontrollprobe umfasst, wobei ein über dem Gehalt an katalytischem Eisen in der Kontrollprobe liegender Gehalt an katalytischem Eisen in der Urinprobe auf eine Nierenerkrankung hinweist; oder

(d) das Verfahren ein Verfahren zur Identifizierung eines an einer progressiven Nierenerkrankung leidenden Menschen umfasst, der von der Behandlung der progressiven Nierenerkrankung mit einem Eisen-Chelatbildner Nutzen zieht, wobei ein Gehalt an katalytischem Eisen in der Urinprobe, der etwa 15 nMol/mg über dem eines Referenzproteins liegt, einen an einer progressiven Nierenerkrankung leidenden Menschen identifiziert, der von der Behandlung der progressiven Nierenerkrankung mit dem Eisen-Chelatbildner Nutzen zieht; oder

(e) in welchem das katalytische Eisen vor oder nach dem Ausbruch klinischer Symptome der Nierenerkrankung gemessen wird.

3. Verwendung eines Eisen-Chelatbildners zur Herstellung eines Medikaments für die Behandlung einer Nierenerkrankung in einem Menschen.

4. Verwendung nach Anspruch 3, bei welcher

(a) der Eisen-Chelatbildner einem Menschen mit ischämischer Nephropathie, einem Menschen mit einer Nierenerkrankung in Folge eines Schadens an einem Niereninterstitium, einem Menschen mit einer progressiven Nierenerkrankung, ausgewählt aus der Gruppe diabetische Nephropathie, primäre Glomerulonephritis und sekundäre Glomerulonephritis verabreicht wird;

(b) der Eisen-Chelatbildner ausgewählt ist aus der Gruppe Deferipron, Deferoxamin, polyanionische Amine und substituierte Polyaza-Verbindungen;

(c) der Eisen-Chelatbildner mit einer Dosis im Bereich zwischen etwa 20 mg/kg Körpergewicht und etwa 150 mg/kg Körpergewicht des Menschen pro Tag verabreicht wird;

(d) der Eisen-Chelatbildner in Mehrfachdosen verabreicht wird;

(e) der Eisen-Chelatbildner oral verabreicht wird;

(f) das Referenzprotein das Kreatinin im Urin ist;

(g) der Proteingehalt in einer von dem Menschen erhaltenen Urinprobe gemessen wird;

(h) der Gehalt an katalytischem Eisen in einer von dem Menschen erhaltenen Urinprobe gemessen wird;

(i) das Kreatinin in einer von dem Menschen erhaltenen Blutprobe gemessen wird;

(j) der Stickstoffgehalt des Blutharnstoffs in einer von dem Menschen erhaltenen Blutprobe gemessen wird;

(k) wobei die Verabreichung des Eisen-Chelatbildners die Verschlechterung der glomerulären Filtrationsrate vermindert; oder

(l) wobei die Verabreichung des Eisen-Chelatbildners einen Vertreter, ausgewählt aus der Gruppe Protein im Urin, Stickstoff im Blutharnstoff, Serum-Kreatinin und Plasma-Kreatinin reduziert.

5. Verwendung nach Anspruch 3. bei welcher der Eisen-Chelatbildner für die Verabreichung an einen Menschen mit einem katalytische Eisengehalt im Urin bestimmt ist, welcher den der Kontrollprobe überschreitet, und die Behandlung ferner die Messung des Gehalts an katalytischem Eisen in einer ersten nach Verabreichung des Eisen-Chelatbildners entnommenen Urinprobe und einen Vergleich des Gehalts an katalytischem Eisen in der ersten Urinprobe mit dem Gehalt an katalytischem Eisen in der Kontrollprobe umfasst.

6. Verwendung nach Anspruch 5, bei welcher die Behandlung ferner nachfolgende Dosen des Eisen-Chelatbildners umfasst, wobei die Menge der nachfolgenden Dosis über einen Vergleich des gemessenen Gehalts an katalytischem Eisen in der Urinprobe mit dem Gehalt an katalytischem Eisen in der Kontrollprobe ermittelt wird, und die Behandlung ferner die Schritte umfasst: Messen des Gehalts an katalytischem Eisen in einer nachfolgenden Urinprobe und Vergleichen des Gehalts an katalytischem Eisen in der nachfolgenden Urinprobe mit dem Gehalt an katalytischem Eisen in der ersten Urinprobe, der zweiten Urinprobe oder beiden; und wobei ferner wahlweise die nachfolgende Dosis des Eisen-Chelatbildners verabreicht wird, bis der Gehalt an katalytischem Eisen im Urin in einer nachfolgenden Urinprobe im Wesentlichen konstant ist; und wobei wahlweise, entweder die nachfolgende Dosis des Eisen-Chelatbildners verabreicht wird bis der im Wesentlichen konstante Gehalt an katalytischem Eisen im Urin in der nachfolgenden Probe etwa gleich dem der Kontrollprobe ist, oder die Dosen des verabreichten Eisen-Chelatbildners in mindestens zwei aufeinander folgenden Dosierungen sich etwa um 10 mg/kg Körpergewicht des Menschen unterscheiden.

**7.** Verwendung nach Anspruch 5, bei welcher

(a) das Kreatinin in einer von dem Menschen erhaltenen Blutprobe gemessen wird;
(b) der Stickstoff-Gehalt des Blutharnstoffs in einer von dem Menschen erhaltenen Blutprobe gemessen wird;
(c) der Eisen-Chelatbildner ausgewählt wird aus der Gruppe Deferipron, Deferoxamin, polyanionische Amine und substituierte Polyaza-Verbindungen;
(c) der Eisen-Chelatbildner mit einer Dosis im Bereich zwischen etwa 20 mg/kg Körpergewicht und etwa 150 mg/kg Körpergewicht des Menschen pro Tag verabreicht wird;
(e) die Urinprobe von einem Menschen mit einer progressiven Nierenerkrankung, ausgewählt aus der Gruppe diabetische Nephropathie, primäre Glomerulonephritis und sekundäre Glomerulonephritis erhalten wird;
(f) die Urinprobe von einem Menschen mit einer progressiven glomerulären Nierenerkrankung erhalten wird;
(g) der Gehalt an katalytischem Eisen in Bezug auf ein Referenzprotein gemessen wird und das Referenzprotein wahlweise das Kreatinin im Urin ist;
(h) die Verabreichung des Eisen-Chelatbildners im Wesentlichen das Fortschreiten der Nierenerkrankung in dem Menschen aufhält;
(i) die Verabreichung des Eisen-Chelatbildners die Schwere einer progressiven Nierenerkrankung in einem Menschen mindert; oder
(j) die Verwendung in der Behandlung einer Mikroalbuminurie besteht und ferner den Schritt der Verabreichung einer nachfolgenden Dosis des Eisen-Chelatbildners umfasst, wobei die Menge der nachfolgenden Dosis über einen Vergleich des gemessenen Gehalts an katalytischem Eisen in der Urinprobe mit dem Gehalt an katalytischem Eisen in der Kontrollprobe ermittelt wird und/oder wobei die Verabreichung des Eisen-Chelatbildners im Wesentlichen das Fortschreiten der Mikroalbuminurie in dem Menschen aufhält.

**8.** Verfahren zur Bewertung der Wirksamkeit der Verabreichung eines Eisen-Chelatbildners zur Behandlung eines unter einer progressiven Nierenerkrankung leidenden Menschen mit dem Schritt des Vergleichs des Gehalts an katalytischem Eisen von einer ersten von dem Menschen erhaltenen Urinprobe mit dem Gehalt an katalytischem Eisen einer nachfolgenden Urinprobe, wobei die nachfolgende Urinprobe erhalten worden ist, nachdem die erste Urinprobe erhalten wurde und nach der Verabreichung des Eisen-Chelatbildners, wobei der Vergleich die Wirksamkeit der Verabreichung des Eisen-Chelatbildners zur Minderung des Fortschreitens der Nierenerkrankung anzeigt.

## Revendications

**1.** Procédé servant à diagnostiquer une maladie du rein chez un être humain comprenant l'étape consistant à mesurer le fer catalytique dans un échantillon d'urine provenant de cet être humain.

**2.** Procédé selon la revendication 1, dans lequel :

(a) le fer catalytique est mesuré par rapport à une protéine de référence dans l'échantillon d'urine, et de manière facultative dans lequel la protéine de référence est de la créatinine urinaire ;
(b) l'échantillon d'urine est prélevé sur un être humain atteint de néphropathie ischémique, une maladie du rein résultant de lésions au niveau d'un interstice d'un rein, une maladie rénale évolutive choisie dans le groupe comprenant la néphropathie diabétique, la glomérulonéphrite primaire et la glomérulonéphrite secondaire, dans lequel la glomérulonéphrite primaire est choisie dans le groupe comprenant la néphropathie membraneuse, la glomérulosclérose segmentaire focale, la néphropathie à IgA, la glomérulosclérose membrano-proliférative, la néphropathie à IgA, la glomérulonéphrite membrano-proliférative et la glomérulonéphrite à croissants épithéliaux, et dans lequel la glomérulonéphrite secondaire est choisie dans le groupe comprenant le lupus érythémateux systémique , le purpura de Henoch-Schönlein et le syndrome hémolytique et urémique ;
(c) le procédé comprend en outre une comparaison de la teneur en fer catalytique dans l'échantillon d'urine avec la teneur en fer catalytique dans un échantillon témoin, dans lequel une teneur en fer catalytique dans l'échantillon d'urine supérieure à la teneur en fer catalytique dans l'échantillon témoin est révélatrice d'une maladie rénale ;

(d) le procédé comprend un procédé consistant à identifier un être humain souffrant d'une maladie rénale évolutive qui bénéficiera du traitement de la maladie rénale évolutive avec un chélateur de fer dans lequel une teneur en fer catalytique dans l'échantillon d'urine supérieure à environ 15 nmol/mg d'une protéine de référence révèle un être humain souffrant d'une maladie rénale évolutive qui bénéficiera du traitement de la maladie rénale évolutive avec le chélateur de fer, ou

(e) dans lequel le fer catalytique est mesuré avant ou après l'apparition des symptômes cliniques de la maladie rénale.

3. Utilisation d'un chélateur de fer pour la fabrication d'un médicament servant à traiter une maladie rénale chez un être humain.

4. Utilisation de la revendication 3 dans laquelle

(a) le chélateur de fer est administré à un être humain atteint de néphropathie ischémique, un être humain atteint de la maladie rénale résultant de lésions au niveau d'un interstice d'un rein, un être humain souffrant d'une maladie rénale évolutive choisie dans le groupe comprenant la néphropathie diabétique, la glomérulonéphrite primaire et la glomérulonéphrite secondaire ;

(b) le chélateur de fer est choisi dans le groupe comprenant la défériprone, la déferoxamine, les amines polyanioniques et les composés polyaza substitués ;

(c) le chélateur de fer est administré par dose d'environ 20 mg/kg du poids corporel à environ 150 mg/kg du poids corporel de l'être humain par jour ;

(d) le chélateur de fer est administré par doses multiples ;

(e) le chélateur de fer est administré par voie orale ;

(f) la protéine de référence est la créatinine urinaire ;

(g) la teneur en protéines dans un échantillon d'urine prélevé sur l'être humain est mesurée ;

(h) la teneur en fer catalytique dans un échantillon d'urine prélevé sur l'être humain est mesurée ;

(i) la créatinine dans un échantillon de sang prélevé sur l'être humain est mesurée ;

(j) la teneur en azote uréique du sang dans un échantillon de sang prélevé sur l'être humain est mesurée ;

(k) dans lequel l'administration du chélateur de fer réduit la détérioration du taux de filtration glomérulaire ; ou

(l) dans lequel l'administration du chélateur de fer réduit un élément choisi dans le groupe comprenant la protéine dans l'urine, l'azote uréique du sang, la créatinine sérique et la créatinine plasmatique.

5. Utilisation de la revendication 3, dans laquelle le chélateur de fer est prévu pour une administration à un être humain ayant une teneur en fer catalytique dans l'urine excédant celle de l'échantillon témoin ; et dans laquelle le traitement comprend en outre la mesure de la teneur en fer catalytique dans un premier échantillon d'urine prélevé après l'administration du chélateur de fer, et la comparaison de la teneur en fer catalytique dans le premier échantillon d'urine avec la teneur en fer catalytique dans l'échantillon témoin.

6. Utilisation de la revendication 5, dans laquelle le traitement comprend en outre des doses suivantes du chélateur de fer, la quantité de la dose suivante étant déterminée en comparant la teneur en fer catalytique mesurée dans l'échantillon d'urine à la teneur en fer catalytique dans l'échantillon témoin, et qui comprend en outre de manière facultative les étapes consistant à

mesurer la teneur en fer catalytique dans un échantillon d'urine ultérieur, et

comparer la teneur en fer catalytique dans l'échantillon d'urine ultérieur à la teneur en fer catalytique dans le premier échantillon d'urine, l'échantillon témoin, ou les deux ;

et encore de manière facultative dans laquelle la dose ultérieure de chélateur de fer est administrée jusqu'à ce que la teneur en fer catalytique urinaire dans un échantillon d'urine ultérieur soit essentiellement constante ;

et de manière facultative soit dans laquelle la dose ultérieure de chélateur de fer est administrée jusqu'à ce que la teneur en fer catalytique urinaire essentiellement constante dans l'échantillon d'urine ultérieur soit à peu près celle de l'échantillon de référence, ou dans laquelle la dose de chélateur de fer administrée dans au moins deux doses successives diffère d'environ 10 mg/kg du poids corporel de l'être humain.

7. Utilisation de la revendication 5, dans laquelle

(a) la créatinine dans un échantillon de sang prélevé sur l'être humain est mesurée ;

(b) la teneur en azote uréique du sang dans un échantillon de sang prélevé sur l'être humain est mesurée ;

(c) le chélateur de fer est choisi dans le groupe comprenant la défériprone, la déferoxamine, les amines polyanioniques et les composés polyaza substitués ;

(d) le chélateur de fer est administré par dose d'environ 20 mg/kg du poids corporel à environ 150 mg/kg du poids corporel de l'être humain par jour ;

(e) l'échantillon d'urine est prélevé sur un être humain souffrant d'une maladie rénale évolutive choisie dans le groupe comprenant la néphropathie diabétique, la glomérulonéphrite primaire et la glomérulonéphrite secondaire ;

(f) l'échantillon d'urine est prélevé sur un être humain atteint d'une maladie glomérulaire rénale évolutive ;

(g) la teneur en fer catalytique est mesurée par rapport à une protéine de référence, et en option la protéine de référence est la créatinine urinaire ;

(h) l'administration du chélateur de fer stoppe essentiellement la progression de la maladie rénale chez l'être humain ;

(i) l'administration du chélateur de fer réduit la gravité de la maladie rénale évolutive chez un être humain ; ou

(j) l'utilisation consiste à traiter la microalbuminurie et en option elle comprend en outre l'étape consistant à administrer une dose suivante du chélateur de fer, la quantité de la dose suivante étant déterminée par la comparaison de la teneur en fer catalytique mesurée dans l'échantillon d'urine avec la teneur en fer catalytique dans l'échantillon témoin, et/ou dans laquelle l'administration du chélateur de fer stoppe essentiellement la progression de la microalbuminurie chez l'être humain.

8. Procédé consistant à évaluer l'efficacité de l'administration d'un chélateur de fer pour traiter un être humain atteint d'une maladie rénale évolutive, comprenant l'étape consistant à comparer la teneur en fer catalytique d'un premier échantillon d'urine prélevé sur l'être humain avec la teneur en fer catalytique d'un échantillon d'urine ultérieur, l'échantillon d'urine ultérieur ayant été prélevé après avoir prélevé le premier échantillon d'urine et après l'administration du chélateur de fer, moyennant quoi la comparaison indique l'efficacité de l'administration du chélateur de fer pour réduire la progression de la maladie rénale.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9401770 A
- DE 4008323
- US 5047421 A
- US 5424057 A
- US 5721209 A
- US 5811127 A
- WO 9605309 A

**Non-patent literature cited in the description**

- **KLAHR, S.** *Contrib. Nephrol.,* 1996, vol. 118, 1-5
- **DE MATTOS, A.M. et al.** Immunologic Renal Diseases. Lippincott-Raven Publishers, 1997, 861-885
- **SIEGEL, N.J. et al.** *Kidney Int.,* 1984, vol. 25, 906-911
- **CRONIN, R.E. et al.** *Am. J. Physiol.,* 1985, vol. 248, F332-F339
- **CRONIN, R.E. et al.** *Am. J. Physiol.,* 1986, vol. 251, F408-F416
- **SEIKEN, G. et al.** *Kidney Int.,* 1994, vol. 45, 1622-1627
- **SHAW, S.G. et al.** *J. Clin. Invest.,* 1987, vol. 80, 1232-1237
- **ALLGREN, R.L. et al.** *N. Eng. J. Med.,* 1997, vol. 336, 828-834
- **PALLER, M..S.** *Sem. Nephrol.,* 1998, vol. 18, 482-489
- **SAVILL, J. et al.** Oxford Textbook of Clinical Nephrology. Oxford Medical Publications, 1998, 403-439
- **HIRSCHBERG, R. et al.** *Kidney Int.,* 1999, vol. 55, 2423-2432
- **LARSEN, K.** *Clin. Chem. Acta.,* 1972, vol. 41, 209-217
- **TALKE, H. et al.** *Klin, Wscr,* 1965, vol. 41, 174
- **FUJITA, Y. et al.** *Bunseki Kgaku,* 1983, vol. 32, 379-386
- **ROLIN, H.A. III et al.** The Principles and Practice of Nephrology. Mosby-Year Book, Inc, 1995, 8-13
- **CARLSON, J.A. et al.** Diseases of the Kidney. Little, Brown and Co., Inc, 1993, 361-405
- **GUTTERIDGE, J.M.C. et al.** *Biochem J.,* 1981, vol. 199, 263-265
- **YERGEY, A.L.** *J. Nutrition,* 1996, vol. 126, 355S-361S
- **IANCU, T.C. et al.** *Biometals,* 1996, vol. 9, 57-65
- **ARTISS, J.D. et al.** *Clin. Biochem.,* 1981, vol. 14, 311-315
- **SMITH, F.E. et al.** *Clin. Biochem.,* 1984, vol. 17, 306-310
- **ARTISS, J.D. et al.** *Microchem. J.,* 1983, vol. 28, 275-284
- **OLIVIERI, N.F. et al.** *New Eng. J. Med.,* 1995, vol. 332, 918-922
- **BOYCE, N.W. et al.** *Kidney International.,* 1986, vol. 30, 813-817
- **KONTOGHIORGHES, G.J.** *Indian J. Pedirr.,* 1993, vol. 60, 485-507
- **HERSHKO, C. et al.** *Brit. J. Haematology,* 1998, vol. 101, 399-406
- **LOWTHER, N. et al.** *Pharmac. Res.,* 1999, vol. 16, 434
- Oxford Textbook of Clinical Nephrology. Oxford University Press, 1998
- Primer on Kidney Diseases. Academic Press, 1998
- **KARLINSKY, M.L. et al.** Kidney Int. 1980, vol. 17, 293-302
- **TOMFORD, R.C. et al.** *J. Clin. Invest.,* 1981, vol. 68, 655-664
- **BALIGA, R. et al.** *Biochem J.,* 1993, vol. 291, 901-905
- **ABBOTT, K.C. et al.** *Arch. Internal Med.,* 1994, vol. 154, 146-153
- **KEEN, H. et al.** *Lancet,* 1968, vol. 2, 913-916
- **SILVER, A. et al.** *Clin. Chem,* 1986, vol. 32, 1303-1306
- **CLOSE, C. et al.** *Diabet. Med.,* 1987, vol. 4, 491-492
- **HARMOINEN, A. et al.** *Clin. Chim. Acta,* 1987, vol. 166, 85-89
- **MARRE, M. et al.** *Clin.. Chem.,* 1987, vol. 33, 209-213
- **MCCORMIK, C.P. et al.** *Ann. Clin. Lab Sci.,* 1989, vol. 19, 944-951
- **CAMBIASO, C.L. et al.** *Clin. Chem.,* 1988, vol. 34, 416-418

- **NIWA, T. et al.** *Clin. Chim. Acta,* 1990, vol. 186, 391-396
- **MATTOCK, M.B. et al.** *Diabetes,* 1992, vol. 41, 736-741
- **NEIL, A. et al.** *Diabetes Care,* 1993, vol. 16, 996-1003
- **ABBOTT, K.C. et al.** *Arch. Intern. Med.,* 1994, vol. 154, 146-153
- Pharmaceutical Sciences. Mack Pub. Co,
- **GUTTERIDGE J.M.C. et al.** *Biochem J,* 1981, vol. 199, 263-265
- **GUTTERIDGE J.M.C. et al.** *Biochem J,* 1981, vol. 199, 263-26
- **KONTOGHIORGHES A.J. et al.** *Inorganica Chimica Acta,* 1987, vol. 136, L11-L12